# EUROPEAN PATENT SPECIFICATION

(11) **EP 1 608 650 B1**
(45) Date of publication and mention of the grant of the patent: **02.06.2010**
(21) Application number: 03719078.2
(22) Date of filing: 31.03.2003
(51) Int. Cl.: C07D 487/04, A61K 31/5517, A61P 35/00

(54) **PYRENE-LINKED PYRROLO(2,1-C)(1,4)BENZODIAZEPINE DERIVATIVES USEFUL AS ANTICANCER AGENTS**
PYREN-GEBUNDENE PYRROLO(2,1-C)(1,4)BENZODIAZEPIN-DERIVATE ZUR VERWENDUNG ALS ANTIKREBSMITTEL
DERIVES DE PYRROLO(2,1-C)(1,4)BENZODIAZEPINE LIES AU PYRENE UTILES EN TANT QU'AGENTS ANTICANCEREUX

(43) Date of publication of application: 28.12.2005
(73) Proprietor: Council of Scientific and Industrial Research, New Delhi 110 001 (IN)
(72) Inventor: KAMAL, Ahmed, Hyderabad 500 007,2Andhra Pradesh (IN); GUJJAR, Ramesh, Hyderabad 500 007, Andhra Pradesh (IN); PODDUTOORI, Ramulu, Hyderabad 500 007, Andhra Pradesh (IN); OLEPU, Srinivas, Hyderabad 500 007, Andhra Pradesh (IN)
(74) Representative: Manaton, Ross Timothy
(86) International application number: PCT/IN2003/000102
(87) International publication number: WO 2004/087711

(56) References cited:
- G. BARALDI ET AL.: "Synthesis, in Vitro Antiproliferative Activity, and DNA-Binding Properties of Hybrid Molecules Containing Pyrrolo(2,1-c)(1,4)benzo- diazepine and Minor-Groove-Binding Oligopyrrole Carriers" JOURNAL OF MEDICINAL CHEMISTRY, vol. 42, no. 25, 1999, pages 5131-5141, XP002272006 cited in the application
- BARALDI P G ET AL: "Design, synthesis and biological activity of a pyrrolo [2,1-c][1,4]benzodiazepine (PBD)-distamycin hybrid" BIOORGANIC & MEDICINAL CHEMISTRY LETTERS, OXFORD, GB, vol. 8, no. 21, 3 November 1998 (1998-11-03), pages 3019-3024, XP004141867 ISSN: 0960-894X
- QUN ZHOU ET AL.: "Design and Synthesis of a Novel DNA-DNA Interstrand Adenine-Guanine Cross-Linking Agent" JOURNAL OF THE AMERICAN CHEMICAL SOCIETY, vol. 123, no. 20, 2001, pages 4865-4866, XP002272007
- B. K. BANIK ET AL.: "Polycyclic Aromatic Compounds as Anticancer Agents: Structure-Activity Relationships of Chrysene and Pyrene Derivatives" BIOORGANIC & MEDICINAL CHEMISTRY, vol. 9, 2001, pages 593-605, XP002272008 cited in the application
- KAMAL A ET AL: "Design, Synthesis, and Evaluation of New Non-Crosslinking Pyrrolobenzodiazepine Dimers with Efficient DNA Binding Ability and Potent Antitumor Activity" JOURNAL OF MEDICINAL CHEMISTRY, AMERICAN CHEMICAL SOCIETY. WASHINGTON, US, vol. 45, 2002, pages 4679-4688, XP002249808 ISSN: 0022-2623 cited in the application
- S. J. GREGSON ET AL.: "Design, Synthesis, and Evaluation of a Novel Pyrrolobenzodiazepine DNA-Interactive Agent with Highly Efficient Cross-Linking Ability and Potent Cytotoxicity" JOURNAL OF MEDICINAL CHEMISTRY, vol. 44, no. 5, 2001, pages 737-748, XP002272009 cited in the application
- D. E. THURSTON ET AL.: "Synthesis of Sequence-Selective C8-Linked Pyrrolo(2,1-c)(1,4)benzodiazepine DNA Interstrand Cross-Linking Agents" JOURNAL OF ORGANIC CHEMISTRY, vol. 61, no. 23, 1996, pages 8141-8147, XP002272010 cited in the application

## Description

### Field of the invention

The present invention relates to a process for the preparation of novel pyrrolo [2,1-*c*][1,4]benzodiazepine hybrids useful as potential antitumour agents. This invention also relates to a process for the preparation of new pyrrolo[2,1-*c*][1,4]benzodiazepine hybrids as potential antitumour agents. More particularly, it provides a process for the preparation of 7-methoxy-8-[N-(1"-pyrenyl)-alkane-3'-carboxamide]-oxy-(11a*S*)-1,2,3,11a-tetraydro 5*H-*pyrrolo[2,1-*c*][1,4]benzodiazepin-5-one, with aliphatic chain length variation of these compounds and it also describes the DNA binding, anticancer (antitumour) activity. The structural formula of this novel pyrrolo[2,1-*c*] [1,4]benzodiazepine is given below:

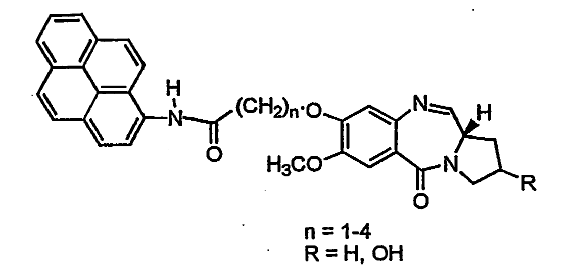

### Background of the invention

Pyrrolo[2,1-*c*][1,4]benzodiazepine antitumour antibiotics are commonly known as anthramycin class of compounds. In the last few years, a growing interest has been shown in the development of new pyrrolo[2,1-*c*][1,4]benzodiazepines (PBDs). These antibiotics react covalently with DNA to form an N2-guanine adduct that lies within the minor groove of duplex DNA *via* an acid-labile aminal bond to the electrophilic imine at the N10-C11 position (Kunimoto, S.; Masuda, T.; Kanbayashi, N.; Hamada, M.; Naganawa, H.; Miyamoto, M.; Takeuchi, T.; and Unezawa, H. J. Antibiot., 1980, 33, 665.; Kohn, K. W. and Speous, C. L. J. Mol. Biol., 1970, 51, 551.; Hurley, L. H.; Gairpla, C. and Zmijewski, M. Biochem. Biophys. Acta., 1977, 475, 521.; Kaplan, D. J. and Hurley, L. H. Biochmestry, 1981, 20, 7572). The molecules have a right-handed twist, which allows them to follow the curvature of the minor groove of B-form double-stranded DNA spanning three base pairs. Recently, PBD dimers have been developed that comprises two C2-exo-methylene substituted DC-81 subunits tethered through their C-8 position via an inert propanedioxy linker (Gregson, S. J.; Howard, P. W.; Hartely, J. A.; Brooks, N. A.; Adams, L. J.; Jerkins, T. C.; Kelland, L. R. and Thurston, D. E. J. Med. Chem. 2001, 44, 737). A recent development has been the linking of two PBD units through their C-8 positions to give bisfunctional alkylating agents capable of cross-linking DNA (Thurston, D. E.; Bose, D. S.; Thomson, A. S.; Howard, P. W.; Leoni, A.; Croker, S. J.; Jenkins, T. C.; Neidle, S. and Hurley, L. H. J. Org. Chem., 1996, 61, 8141).

Recently, a noncross-linking mixed imine-amide PBD dimers have been synthesized that have significant DNA binding ability and potent anti tumour activitiy. (Kamal, A.; Ramesh, G.; Laxman, N.; Ramulu, P.; Srinivas, O.; Neelima, K.; Kondapi, A. K.; Srinu, V. B.; Nagarajaram, H. M. J. Med. Chem. 2002, 45, 4679).

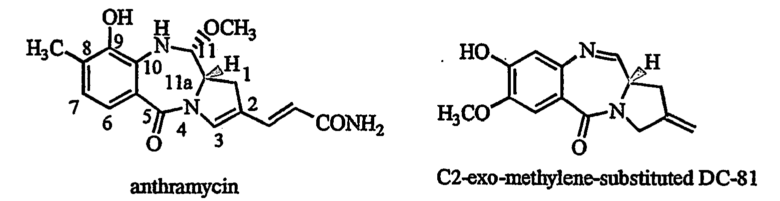

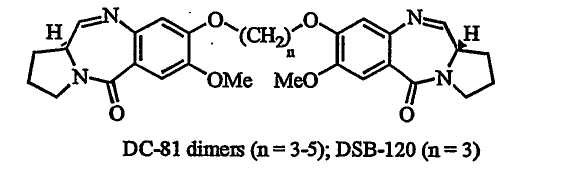

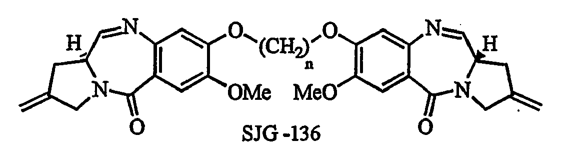

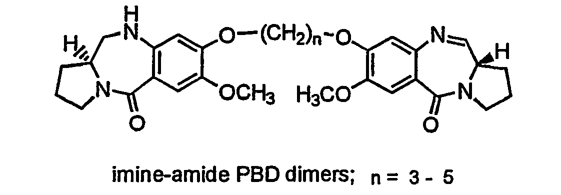

Naturally occurring pyrrolo[2,1-*c*][1,4]benzodiazepines belong to a group of antitumour antibiotics derived from *Streptomyces* species. Recently, there is much impetus for the PBD systems as they can recognize and bind to specific sequence of DNA. Examples of naturally occurring PBD's include anthramycin, DC-81, tomaymycin, sibiromycin and neothramycin. However, the clinical efficacy for these antibiotics is hindered by several limitations, such as poor water solubility, cardiotoxicity, development of drug resistance and metabolic inactivation.

### Objects if the invention

The main object of the present invention is to provide new pyrrolo[2,1-*c*][1,4]-benzodiazepine hybrids useful as antitumour agents.

Another objective of the present invention is to provide a process for the preparation of novel pyrrolo[2,1-*c*][1,4]-benzodiazepine hybrids useful as antitumour agents.

### Summary of the invention

Accordingly the present invention provides pyrrolo[2,1-*c*][1,4]benzodiazepine hybrids of formula V wherein R = H, OH and n is 1-4

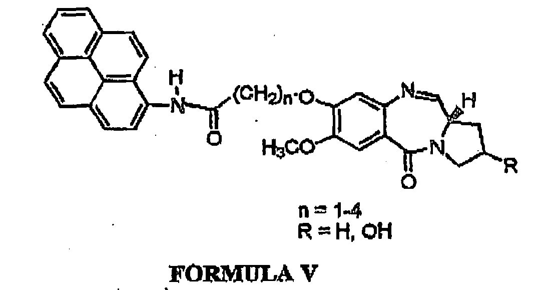

Accordingly the present invention provides a process for preparation of pyrrolo[2,1-*c*][1,4]benzodiazepine hybrids of formula V 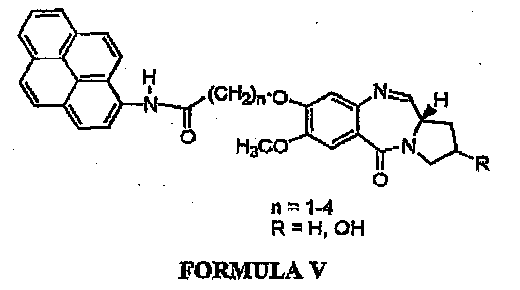 which comprises reacting pyrene amine of formula I 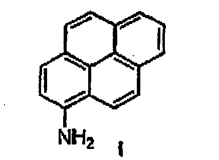 with (2*S*)-N-{4-[(3'-carboxy alkyl)oxy]-5-methoxy-2-nitrobenzoyl}pyrrolidine-2-carboxaldehyde diethyl thioacetal of formula II where R is as stated above 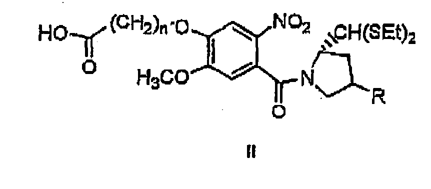 in the presence of isobutyl chloroformate, bases like triethyl amine, DBU in presence of organic solvents up to refluxing for a period of 24 h isolating (2*S*)-N-{4-[N-(1"-pyrenyl)-alkane-3'-carboxamide]-oxy--5-methoxy-2-nitrobenzoyl}pyrrolidine-2-carboxaldehyde diethyl thioacetal III where n is 1-4 and R is as stated above by conventional methods, 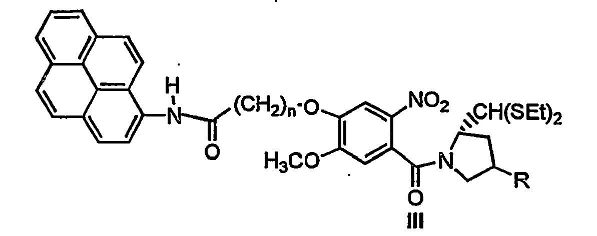 reducing the above nitro compounds of formula III with SnCl₂.2H₂O in presence of organic solvent up to a reflux temperature, isolating the (2*S*)-N-{4-[N-(1"-pyrenyl)-alkane-3'-carboxamide]-oxy--5-methoxy-2-aminobenzoyl}pyrrolidine-2-carboxaldehyde diethyl thioacetal of formula IV where n is 1-4 and R is as stated above by known methods, 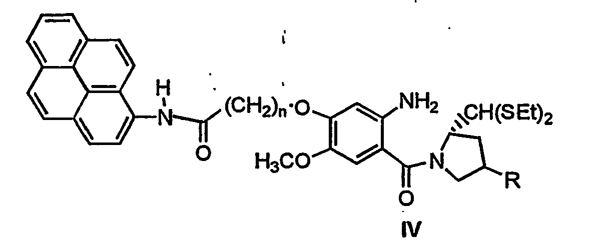 reacting the above said amino compound of formula IV with known deprotecting agents in a conventional manner to give novel pyrrolo[2,1-*c*][1,4]benzodiazepine hybrids of formula V wherein n and R are as stated above.

### Detailed description of the invention

The precursors, pyrene amine of formula I (Banik, B. K.; Becker, F. F. Bioorg. Med Chem. 2001, 9, 593) and (2*S*)-N-{4-[(3'-carboxy alkyl)oxy]-5-methoxy-2-nitrobenzoyl} pyrrolidine-2-carboxaldehyde diethyl thioacetal of formula II (Baraldi, P. G.; Balboni, G.; Cacciari, B.; Guiotto, A.; Manfredini, S.; Romagnoli, R.; Spalluto, G.; Thurston, D. E.; Howard, P. W.; Bianchi, N.; Rutigiiano, C.; Mischiati, C. and Gambari, R. J. Med. Chem. 1999, 42, 5131.; Reddy, B. S. P.; Damayanthi, Y.; Reddy, B. S. N.; Lown, W. J. Anti-Cancer Drug Design 2000, 15, 225) have been prepared by literature methods.

These new analogues of pyrrolo[2,1-*c*][1,4]benzodiazepine hybrids linked at C-8 position have shown promising DNA binding activity and efficient anticancer activity in various cell lines. The molecules synthesized are of immense biological significance with potential sequence selective DNA-binding property. This resulted in design and synthesis of new congeners as illustrated in Scheme-1, which comprise:
1. The ether linkage at C-8 position of DC-81 intermediates with pyrene ring moiety.
2. Refluxing the reaction mixture for 24-48 h.
3. Synthesis of C-8 linked PBD antitumour antibiotic hybrid imines.
4. Purification by column chromatography using different solvents like ethyl acetate, hexane, dichloromethane and methanol.

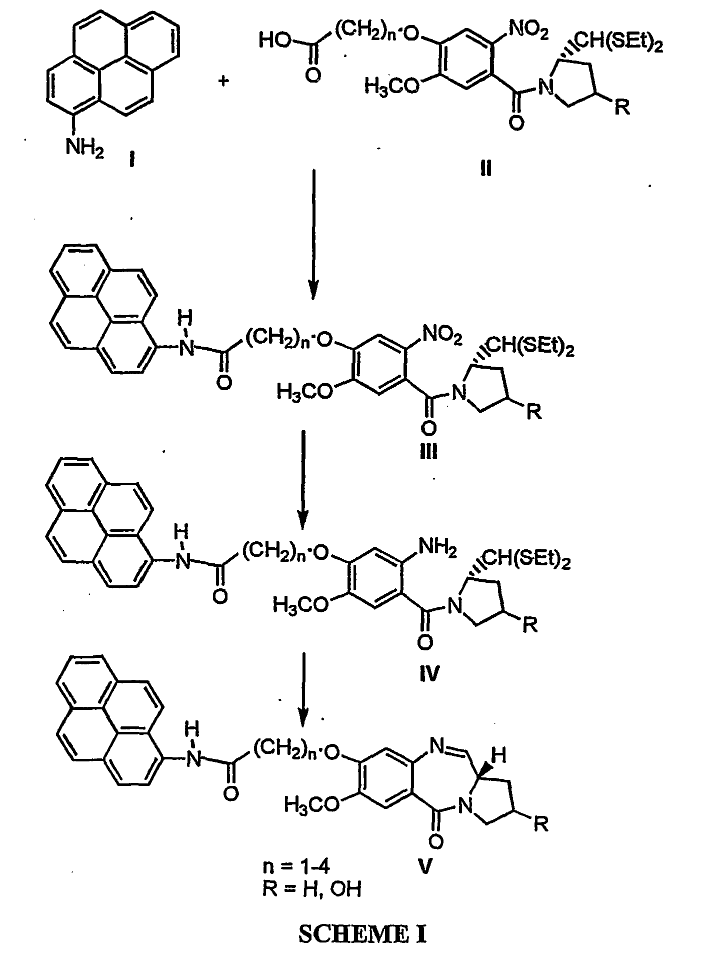

Some representative compounds of formula V present invention are given below
1. 7-Methoxy-8-[N-(1"-pyrenyl)-methane-1'-carboxamide]-oxy-(11a*S*)1,2,3,11a tetrahydro-5*H*-pyrrolo[2,1-*c*][1,4]benzodiazepin-5-one
2. 7-Methoxy-8-[N-(1"-pyrenyl)-methane-1'-carboxamide]-oxy-(4*R*)-hydroxy (11a*S*) 1,2,3,11a tetrahydro-5*H*-pyrrolo[2,1-*c*][1,4]benzodiazepin-5-one
3. 7-Methoxy-8-[N-(1"-pyrenyl)-athane-2'-carboxamidel-oxy-(11a*S*)1,2,3,11a tetrahydro-5*H*-pyrrolo[2,1-*c*][1,4]benzodiazepin-5-one
4. 7-Methoxy-8-[N-(1"-pyrenyl)-ethane-2'-carboxamide]-oxy-(4*R*)-hydroxy (11a*S*) 1,2,3,11a tetrahydro-5*H*-pyrrolo[2,1-*c*][1,4]benzodiazepin-5-one
5. 7-Methoxy-8-[N-(1"-pyrenyl)-propane-3'-carboxamide]-oxy-(11a*S*)-1,2,3,11a tetra-hydro-5*H*-pyrrolo[2,1-*c*][1,4]benzodiazepin-5-one
6. 7-Methoxy-8-[N-(1"-pyrenyl)-propane-3'-carboxamide]-oxy-(4*R*)-hydroxy (11a*S*)-1,2,3,11a-tetra-hydro-5*H*-pyrrolo[2,1-*c*][1,4]benzodiazepin-5-one
7. 7-Methoxy-8-[N-(1"-pyrenyl)-butane-4'-carboxamide]-oxy-(11a*S*)-1,2,3,11a-tetrahydro-5*H*-pyrrolo[2,1-*c*][1,4]benzodiazepin-5-one
8. 7-Methoxy-8-[N-(1"-pyrenyl)-butane-4'-carboxamide]-oxy-(4*R*)-hydroxy (11a*S*)-1,2,3,11a-tetra-hydro-5*H*-pyrrolo[2,1-*c*][1,4]benzodiazepin-5-one

The following examples are given by way of illustration.

### Example 1

Compound (2*S*)-N-[4-[(1'-carboxy methyl)oxy]-5-methoxy-2-nitrobenzoyl] pyrrolidine-2-carboxaldehyde diethyl thioacetal of formula II (2.29 g, 5 mmol) was taken in dry CH₂Cl₂ (20 mL), TEA (707 mg, 7 mmol) was added and the mixture was cooled at 0-5 °C. Isobutyl chloroformate (819 mg, 6 mmol) in dry CH₂Cl₂ (10 mL) was added dropwise and the mixture was kept at 0-5 °C for 15 min. A solution of 1-amino pyrene of formula 1 (251 mg, 5 mmol) in CH₂Cl₂ was added to it at the same temperature and the solution was stirred at room temperature for overnight. The mixture was washed with saturated NaHCO₃ (50 mL), brine, dried and solvent was evaporated. The crude material was chromatographed over silica gel using ethyl acetate/hexane (8:2) solvent to give compound (2*S*)-N-{4-[N-(1"-pyrenyl)-methane-3'-carboxamide]-oxy-5-methoxy-2-nitro-benzoyl}pyrrolidine-2-carboxaldehyde diethyl thioacetal of formula III as a yellow liquid.

The (2*S*)-N-{4-[N-(1"-pyrenyl)-methane-1'-carboxamide]-oxy-5-methoxy-2-nitro benzoyl}pyrrolidine-2-carboxodehyde diethyl thioacetal of formula III (0.657 g, 1 mmol) was dissolved in ethyl acetate (15 mL) and added SnCl₂.2H₂O (1.12 g, 5 mmol) was refluxed for 3 h or until the TLC indicated that reaction was completed. The reaction mixture was then adjusted to pH 8 carefully with saturated NaHCO₃ solution, diluted with ethyl acetate, filtered through celite and extracted. The combined organic phase was dried over Na₂SO₄, and evaporated under vacuum to afford the crude (2*S*)-N-{4-[N-(1"-pyrenyl)-methane-1'-carboxamide]-oxy--5-methoxy-2-aminobenzoyl}pyrrolidine-2-carboxaldehyde diethyl thioacetal of formula IV.

A solution of (2*S*)-N-{4-[N-(1"-pyrenyl)-methane-1'-carboxamide]-oxy-5-methoxy-2-aminobenzoyl}pyrrolidine-2-carboxaldehyde diethyl thioacetal of formula IV (627 mg, 1 mmol), HgCl₂ (613 mg, 2.26 mmol) and CaCO₃ (246 mg, 2.46 mmol) in MeCN-water (4:1) was stirred slowly at room temperature until TLC indicates complete loss of starting material. The reaction mixture was diluted with EtOAc (30 mL) and filtered through a celite bed. The clear yellow organic supernatant was extracted with saturated 5% NaHCO₃ (20 mL), brine (20 mL) and the combined organic phase is dried (Na₂SO₄). The organic layer was evaporated in vacuum and purified by column chromatography (90% CH₂Cl₂-MeOH) to give compound 7-Methoxy-8-[N-(1"-pyrenyl)-propane-3'-carboxamide]-oxy-(11a*S*)-1,2,3,11a tetra-hydro-5*H*-pyrrolo[2,1-*c*][1,4]benzo-diazepin-5-one as pale yellow oil.

### Example 2

Compound (4*R*)-hydroxy-(2*S*)-N-[4-[(1'-carboxy methyl)oxy]-5-methoxy-2-nitrobenzoyl]pyrrolidine-2-carboxaldehyde diethyl thioacetal of formula II (2.37 g, 5 mmol) was taken in dry CH₂Cl₂ (20 mL), TEA (707 mg, 7 mmol) was added and the mixture was cooled at 0-5 °C. Isobutyl chloroformate (819 mg, 6 mmol) in dry CH₂Cl₂ (10 mL) was added dropwise and the mixture was kept at 0-5 °C for 15 min. A solution of 1-amino pyrene formula I (251 mg, 5 mmol) in CH₂Cl₂ was added to it at the same temperature and the solution was stirred at room temperature for overnight. The mixture was washed with saturated NaHCO₃ (50 mL), brine, dried and solvent was evaporated. The crude material was chromatographed over silica gel using ethyl acetate/hexane (8:2) solvent to give compound (2*S*)-N-{4-[N-(1"-pyrenyl)-methane-1'-carboxamide]-oxy--5-methoxy-2-nitrobenzoyl} pyrrolidine-2-carboxaldehyde diethyl thioacetal of formula III as a yellow liquid.

The (4*R*)-hydroxy-(2*S*)-N-{4-[N-(1"-pyrenyl)-methane-1'-carboxamide]-oxy--5-methoxy-2-nitrobenzoyl}pyrrolidine-2-carboxaldehyde diethyl thioacetal of formula III (0.673 g, 1 mmol) was dissolved in ethyl acetate (15 mL) and added SnCl₂.2H₂O (1.12 g, 5 mmol) was refluxed for 3 h or until the TLC indicated that reaction was completed. The reaction mixture was then adjusted to pH 8 carefully with saturated NaHCO₃ solution, diluted with ethyl acetate, filtered through celite and extracted. The combined organic phase was dried over Na₂SO₄, and evaporated under vacuum to afford the crude (4*R*)-hydroxy-(2*S*)-N-{4-[N-(1"-pyrenyl)-methane-1'-carboxamide]-oxy--5-methoxy-2-amino-benzoyl}pyrrolidine-2-carboxaldehyde diethyl thioacetal of formula IV.

A solution of (4*R*)-hydroxy-(2*S*)-N-{4-[N-(1"-pyrenyl)-methane-1'-carboxamide]-oxy--5-methoxy-2-aminobenzoyl}pyrrolidine-2-carboxaldehyde diethyl thioacetal of formula IV (643 mg, 1 mmol), HgCl₂ (613 mg, 2.26 mmol) and CaCO₃ (246 mg, 2.46 mmol) in MeCN-water (4:1) was stirred slowly at room temperature until TLC indicates complete loss of starting material. The reaction mixture was diluted with EtOAc (30 mL) and filtered through a celite bed. The clear yellow organic supernatant was extracted with saturated 5% NaHCO₃ (20 mL), brine (20 mL) and the combined organic phase is dried (Na₂SO₄). The organic layer was evaporated in vacuum and purified by column chromatography (90% CH₂Cl₂-MeOH) to give compound 7-methoxy-8-[N-(1"-pyrenyl)-Methane-1'-carboxamide]-oxy-(4*R*)-hydroxy-(11a*S*)-1,2,3,11atetra-hydro-5*H*-pyrrolo[2,1-*c*][1,4]benzodiazepin-5-one as pale yellow oil.

### Example 3

Compound (2*S*)-N-[4-[(2'-carboxy ethyl)oxy]-5-methoxy-2-nitrobenzoyl] pyrrolidine-2-carboxaldehyde diethyl thioacetal of formula II (2.36 g, 5 mmol) was taken in dry CH₂Cl₂ (20 mL), TEA (707 mg, 7 mmol) was added and the mixture was cooled at 0-5 °C. Isobutyl chloroformate (819 mg, 6 mmol) in dry CH₂Cl₂ (10 mL) was added dropwise and the mixture was kept at 0-5 °C for 15 min. A solution of 1-amino pyrene of formula I (251 mg, 5 mmol) in CH₂Cl₂ was added to it at the same temperature and the solution was stirred at room temperature for overnight. The mixture was washed with saturated NaHCO₃ (50 mL), brine, dried and solvent was evaporated. The crude material was chromatographed over silica gel using ethyl acetate/hexane (8:2) solvent to give compound (2*S*)-N-{4-[N-(1"-pyrenyl)-ethane-2'-carboxamide]-oxy--5-methoxy-2-nitro-benzoyl}pyrrolidine-2-carboxaldehyde diethyl thioacetal of formula III as a yellow liquid.

The (2*S*)-N-{4-[N-(1"-pyrenyl)-ethane-2-carboxamide]-oxy--5-methoxy-2-nitro benzoyl}pyrrolidine-2-carboxaldehyde diethyl thioacetal of formula III (0.671 g, 1 mmol) was dissolved in ethyl acetate (15 mL) and added SnCl₂.2H₂O (1.12 g, 5 mmol) was refluxed for 3 h or until the TLC indicated that reaction was completed. The reaction mixture was then adjusted to pH 8 carefully with saturated NaHCO₃ solution, diluted with ethyl acetate, filtered through celite and extracted. The combined organic phase was dried over Na₂SO₄, and evaporated under vacuum to afford the crude (2*S*)-N-{4-[N-(1"-pyrenyl)-ethane-2'-carboxamide]-oxy--5-methoxy-2-aminobenzoyl}pyrrolidine-2-carboxaldehyde diethyl thioacetal of formula IV.

A solution of (2*S*)-N-{4-[N-(1"-pyrenyl)-ethane-2'-carboxamide]-oxy--5-methoxy-2-aminobenzoyl}pyrrolidine-2-carboxaldehyde diethyl thioacetal of formula IV (641 mg, 1 mmol), HgCl₂ (613 mg, 2.26 mmol) and CaCO₃ (246 mg, 2.46 mmol) in MeCN-water (4:1) was stirred slowly at room temperature until TLC indicates complete loss of starting material.
The reaction mixture was diluted with EtOAc (30 mL) and filtered through a celite bed. The clear yellow organic supernatant was extracted with saturated 5% NaHCO₃ (20 mL), brine (20 mL) and the combined organic phase is dried (Na₂SO₄). The organic layer was evaporated in vacuum and purified by column chromatography (90% CH₂Cl₂-MeOH) to give compound 7-Methoxy-8-[N-(1"-pyrenyl)-ethane-2-carboxamide]-oxy-(11a*S*)-1,2,3,11atetrahydro-5*H*-pyrrolo[2,1-*c*][1,4]benzodiazepin-5-one as pale yellow oil.

### Example 4

Compound (4*R*)-hydroxy-(2*S*)-N-[4-[(2'-carboxy ethyl)oxy]-5-methoxy-2-nitrobenzoyl] pyrrolidine-2-carboxaldehyde diethyl thioacetal of formula II (2.44 g, 5 mmol) was taken in dry CH₂Cl₂ (20 mL), TEA (707 mg, 7 mmol) was added and the mixture was cooled at 0-5 °C. Isobutyl chloroformate (819 mg, 6 mmol) in dry CH₂Cl₂ (10 mL) was added dropwise and the mixture was kept at 0-5 °C for 15 min. A solution of 1-amino pyrene of formula I (251 mg, 5 mmol) in CH₂Cl₂ was added to it at the same temperature and the solution was stirred at room temperature for overnight. The mixture was washed with saturated NaHCO₃ (50 mL), brine, dried and solvent was evaporated. The crude material was chromatographed over silica gel using ethyl acetate/hexane (8:2) solvent to give compound (4*R*)-hydroxy-(2*S*)-N-{4-[N-(1"-pyrenyl)-ethane-2'-carboxamide]-oxy--5-methoxy-2-nitrobenzoyl)pyrrolidine-2-carboxaldehyde diethyl thioacetal of formula III as a yellow liquid.

The(4*R*)-hydroxy-(2*S*)-N-{4-[N-(1"-pyrenyl)-ethane-2'-carboxamide]-oxy-5-methoxy -2-nitrobenzoyl}pyrrolidine-2-carboxaldehyde diethyl thioacetal of formula III (0.687 g, 1 mmol) was dissolved in ethyl acetate (15 mL) and added SnCl₂.2H₂O (1.12 g, 5 mmol) was refluxed for 3 h or until the TLC indicated that reaction was completed. The reaction mixture was then adjusted to pH 8 carefully with saturated NaHCO₃ solution, diluted with ethyl acetate, filtered through celite and extracted. The combined organic phase was dried over Na₂SO₄, and evaporated under vacuum to afford the crude (4*R*)-hydroxy-(2*S*)-N-{4-[N-(1"-pyrenyl)-ethane-2'-carboxamide]-oxy--5-methoxy-2-amino-benzoyl}pyrrolidine-2-carboxaldehyde diethyl thioacetal of formula IV.

A solution of (4*R*)-hydroxy-(2*S*)-N-{4-[N-(1"-pyrenyl)-ethane-2'-carboxamide]-oxy-5-methoxy-2-aminobenzoyl}pyrrolidine-2-carboxaldehyde diethyl thioacetal of formula IV (657 mg, 1 mmol), HgCl₂ (613 mg, 2.26 mmol) and CaCO₃ (246 mg, 2.46 mmol) in MeCN-water (4:1) was stirred slowly at room temperature until TLC indicates complete loss of starting material. The reaction mixture was diluted with EtOAc (30 mL) and filtered through a celite bed. The clear yellow organic supernatant was extracted with saturated 5% NaHCO₃ (20 mL), brine (20 mL) and the combined organic phase is dried (Na₂SO₄). The organic layer was evaporated in vacuum and purified by column chromatography (90% CH₂Cl₂-MeOH) to give compound 7-Methoxy-8-[N-(1"-pyrenyl)-ethane-2'-carboxamide]-oxy-(4*R*)-hydroxy-(11a*S*)-1,2,3,11atetra-hydro-5*H*-pyrrolo[2,1-*c*][1,4]benzodiazepin-5-one as pale yellow oil.

### Example 5

Compound (2*S*)-N-[4-[(3-carboxy propyl)oxy]-5-methoxy-2-nitrobenzoyl] pyrrolidine-2-carboxaldehyde diethyl thioacetal of formula II (2.43 g, 5 mmol) was taken in dry CH₂Cl₂ (20 mL), TEA (707 mg, 7 mmol) was added and the mixture was cooled at 0-5°C. Isobutyl chloroformate (819 mg, 6 mmol) in dry CH₂Cl₂ (10 mL) was added dropwise and the mixture was kept at 0-5 °C for 15 min. A solution of 1-amino pyrene formula I (251 mg, 5 mmol) in CH₂Cl₂ was added to it at the same temperature and the solution was stirred at room temperature for overnight. The mixture was washed with saturated NaHCO₃ (50 mL), brine, dried and solvent was evaporated. The crude material was chromatographed over silica gel using ethyl acetate/hexane (8:2) solvent to give compound (2*S*)-N-{4-[N-(1"-pyrenyl)-propane-3'-carboxamide]-oxy-5-methoxy-2-nitrobenzoyl}pyrrolidine-2-carboxaldehyde diethyl thioacetal of formula III as a yellow liquid (1.92 g, 56%).
¹H NMR (CDCl₃) δ 1.10-1.40 (m, 6H), 1.40-2.40 (m, 6H), 2.50-2.90 (m, 4H), 3.10-3.25 (m, 2H), 3.60 (s, 3H), 4.0-4.20(m, 2H), 4.55-4.85 (m, 2H), 6.70 (s, 1H), 7.62 (s, 1H), 7.70-8.40 (m, 9H), 8.60-8.90 (m, 1H); MS (FAB) 686 [M + H]⁺.

The (2*S*)-N-{4-[N-(1"-pyrenyl)-propane-3'-carboxamide]-oxy--5-methoxy-2-nitro benzoyl}pyrrolidine-2-carboxaldehyde diethyl thioacetal of formula III (0.685 g, 1 mmol) was dissolved in ethyl acetate (15 mL) and added SnCl₂.2H₂O (1.12 g, 5 mmol) was refluxed for 3 h or until the TLC indicated that reaction was completed. The reaction mixture was then adjusted to pH 8 carefully with saturated NaHCO₃ solution, diluted with ethyl acetate, filtered through celite and extracted. The combined organic phase was dried over Na₂SO₄, and evaporated under vacuum to afford the crude (2*S*)-N-{4-[N-(1"-pyrenyl)-propane-3'-carboxamide]-oxy-5-methoxy-2-aminobenzoyl}pyrrolidine-2-carboxaldehyde diethyl thioacetal of formula IV (458 mg, 70%).
¹H NMR (CDCl₃) δ 1.10-1.40 (m, 6H), 1.50-2.30 (m, 8H), 2.40-2.80 (m, 4H), 3.40 (s, 3H), 3.45-3.60 (m, 2H), 4.05-4.15 (m, 2H), 4.50-4.70 (m, 2H), 6.25 (s, 1H), 6.70 (s, 1H), 7.65-8.30 (m, 9H), 9.10-9.25 (m, 1H).

A solution of (2*S*)-N-{4-[N-(1"-pyrenyl)-propane-3'-carboxamide]-oxy--5-methoxy-2-aminobenzoyl}pyrrolidine-2-carboxaldehyde diethyl thioacetal of formula IV (655 mg, 1 mmol), HgCl₂ (613 mg, 2.26 mmol) and CaCO₃ (246 mg, 2.46 mmol) in MeCN-water (4:1) was stirred slowly at room temperature until TLC indicates complete loss of starting material. The reaction mixture was diluted with EtOAc (30 mL) and filtered through a celite bed. The clear yellow organic supernatant was extracted with saturated 5% NaHCO₃ (20 mL), brine (20 mL) and the combined organic phase is dried (Na₂SO₄). The organic layer was evaporated in vacuum and purified by column chromatography (90% CH₂Cl₂-MeOH) to give compound 7-Methoxy-8-[N-(1"-pyrenyl)-propane-3'-carboxamide]-oxy-(11a*S*)-1,2,3,11atetra-hydro-5*H*-pyrrolo[2,1-*c*][1,4]benzodiazepin-5-one as pale yellow oil of formula V (285 mg, 54%).
¹H NMR (CDCl₃) δ 1.40-2.40 (m, 10H), 2.60-2.90 (m, 2H), 3.40-4.05 (m, 4H), 4.10-4.40 (m, 2H), 6.85 (s, 1H), 7.40 (s, 1H), 7.65 (d, 1H), 7.75-8.20 (m, 8H), 8.20-8.40 (m, 1H), 9.0-9.10 (m, 1H); MS (FAB) 530 [M + H]⁺.

### Example 6

Compound (4*R*)-hydroxy-(2*S*)-N-[4-[(3'-carboxy propyl)oxy]-5-methoxy-2-nitrobenzoyl] pyrrolidine-2-carboxaldehyde diethyl thioacetal of formula II (2.51 g, 5 mmol) was taken in dry CH₂Cl₂ (20 mL), TEA (707 mg, 7 mmol) was added and the mixture was cooled at 0-5 °C. Isobutyl chloroformate (819 mg, 6 mmol) in dry CH₂Cl₂ (10 mL) was added dropwise and the mixture was kept at 0-5 °C for 15 min. A solution of 1-amino pyrene of formula I (251 mg, 5 mmol) in CH₂Cl₂ was added to it at the same temperature and the solution was stirred at room temperature for overnight. The mixture was washed with saturated NaHCO₃ (50 mL), brine, dried and solvent was evaporated. The crude material was chromatographed over silica gel using ethyl acetate/hexane (8:2) solvent to give compound (4*R*)-hydroxy-(2*S*)-N-{4-[N (1"-pyrenyl)-propane-3'-carboxamide]-oxy--5-methoxy-2-nitrobenzoyl}pyrrolidine-2-carboxaldehyde diethyl thioacetal of formula III as a yellow liquid.

The (4*R*)-hydroxy-(2*S*)-N-{4-[N-(1"-pyrenyl)-propane-3'-carboxamide]-oxy--5-methoxy-2-nitrobenzoyl}pyrrolidine-2-carboxaldehyde diethyl thioacetal of formula III (0.701 g, 1 mmol) was dissolved in ethyl acetate (15 mL) and added SnCl₂.2H₂O (1.12 g, 5 mmol) was refluxed for 3 h or until the TLC indicated that reaction was completed. The reaction mixture was then adjusted to pH 8 carefully with saturated NaHCO₃ solution, diluted with ethyl acetate, filtered through celite and extracted. The combined organic phase was dried over Na₂SO₄, and evaporated under vacuum to afford the crude (4*R*)-hydroxy-(2*S*)-N-{4-[N-(1"-pyrenyl)-propane-3'-carboxamide]-oxy--5-methoxy-2-aminobenzoyl}pyrrolidine-2-carboxaldehyde diethyl thioacetal of formula IV.

A solution of (4*R*)-hydroxy-(2*S*)-N-{4-[N-(1"-pyrenyl)-propane-3'-carboxamide]-oxy--5-methoxy-2-aminobenzoyl}pyrrolidine-2-carboxaldehyde diethyl thioacetal of formula IV (671 mg, 1 mmol), HgCl₂ (613 mg, 2.26 mmol) and CaCO₃ (246 mg, 2.46 mmol) in MeCN-water (4:1) was stirred slowly at room temperature until TLC indicates complete loss of starting material. The reaction mixture was diluted with EtOAc (30 mL) and filtered through a celite bed. The clear yellow organic supernatant was extracted with saturated 5% NaHCO₃ (20 mL), brine (20 mL) and the combined organic phase is dried (Na₂SO₄). The organic layer was evaporated in vacuum and purified by column chromatography (90% CH₂Cl₂-MeOH) to give compound 7-Methoxy-8-[N-(1"-pyrenyl)-propane-3'-carboxamide]-oxy-(4*R*) hydroxy-(11a*S*)-1,2,3,11atetra-hydro-5*H*-pyrrolo[2,1-*c*][1,4]benzodiazepin-5-one as pale yellow oil of formula V.

### Example 7

Compound (2*S*)-N-{4-[(3'-carboxy butyl)oxy]-5-methoxy-2-nitrobenzoyl}pyrrolidine -2-car-boxaldehyde diethyl thioacetal of formula II (2.50 g, 5 mmol) was taken in dry CH₂Cl₂ (20 mL), TEA (707 mg, 7 mmol) was added and the mixture was cooled at 0-5 °C. Isobutyl chloroformate (819 mg, 6 mmol) in dry CH₂Cl₂ (10 mL) was added dropwise and the mixture was kept at 0-5 °C for 15 min. A solution of 1-amino pyrene of formula I (251 mg, 5 mmol) in CH₂Cl₂ was added to it at the same temperature and the solution was stirred at room temperature for overnight. The mixture was washed with saturated NaHCO₃ (50 mL), brine, dried and solvent was evaporated. The crude material was chromatographed over silica gel using ethyl acetate/hexane (8:2) solvent to give compound (2*S*)-N-{4-[N-(1"-pyrenyl)-butane-3'-carboxamide]-oxy-5-methoxy-2-nitrobe-nzoyl}pyrrolidine-2-carboxaldehyde diethyl thioacetal of formula III as a yellow liquid (1.92 g, 55%).
¹H NMR (CDCl₃) δ 1.10-1.40 (m, 6H), 1.40-2.40 (m, 8H), 2.50-2.90 (m, 4H), 3.10-3.25 (m, 2H), 3.60 (s, 3H), 4.0-4.20 (m, 2H), 4.55-4.85 (m, 2H), 6.70 (s, 1H), 7.62 (s, 1H), 7.70-8.40 (m, 9H), 8.60-8.90 (m, 1H); MS (FAB) 700 [M + H]⁺.

The nitro diethyl thioacetal (2*S*)-N-{4-[N-(1"-pyrenyl)-butane-3'-carboxamide]-oxy-5-methoxy-2-nitrobenzo-yl}pyrrolidine-2-carboxaldehyde diethyl thioacetal of formula III (0.699 g, 1 mmol) was dissolved in ethyl acetate (15 mL) and added SnCl₂.2H₂O (1.12 g, 5 mmol) was refluxed for 3 h or until the TLC indicated that reaction was completed. The reaction mixture was then adjusted to pH 8 carefully with saturated NaHCO₃ solution, diluted with ethyl acetate, filtered through celite and extracted. The combined organic phase was dried over Na₂SO₄, and evaporated under vacuum to afford the crude amino diethyl thioacetal (2*S*)-N-{4-[N-(1"-pyrenyl)-butane-3'-carboxamide]-oxy-5-methoxy-2-aminobenzoyl} pyrrolidine-2-carboxaldehyde diethyl thioacetal of formula IV (482 mg, 72%).
¹H NMR (CDCl₃) δ 1.10-1.40 (m, 6H), 1.50-2.30 (m, 10H), 2.40-2.80 (m, 6H), 3.40 (s; 3H), 3.45-3.60 (m, 2H), 4.05-4.15 (m, 2H), 4.50-4.70 (m, 2H), 6.25 (s, 1H), 6.70 (s, 1H), 7.65-8.30 (m, 9H), 9.10-9.25 (m, 1H).

A solution of (2*S*)-N-{4-[N-(1"-pyrenyl)-butane-3'-carboxamide]-oxy-5-methoxy-2-aminobenzoyl}pyrrolidine-2-carboxaldehyde diethyl thioacetal of formula IV (669 mg, 1 mmol), HgCl₂ (613 mg, 2.26 mmol) and CaCO₃ (246 mg, 2.46 mmol) in MeCN-water (4:1) was stirred slowly at room temperature until TLC indicates complete loss of starting material. Reaction mixture was diluted with EtOAc (30 mL) and filtered through a celite bed. The clear yellow organic supernatant was extracted with saturated 5% NaHCO₃ (20 mL), brine (20 mL) and combined organic phase is dried (Na₂SO₄). The organic layer was evaporated in vacuum and purified by column chromatography (90% CH₂Cl₂-MeOH) to give compound 7-Methoxy-8-[N-(1"-pyrenyl)-butne-4'-carboxamide]-oxy-(11a*S*)-1,2,3,11a-tetra-hydro-5*H-*pyrrolo[2,1-*c*][1,4]benzodiazepin-5-one of formula V as pale yellow oil (266 mg, 49%).
¹H NMR (CDCl₃) δ 1.40-2.40 (m, 12H), 2.60-2.90 (m, 2H), 3.40-4.05 (m, 4H), 4.10-4.40 (m, 2H), 6.85 (s, 1H), 7.40 (s, 1H), 7.65 (d, 1H), 7.75-8.20 (m, 8H), 8.20-8.40 (m, 1H), 9.0-9.10 (m, 1H); MS (FAB) 544 [M + H]⁺.

### Example 8

Compound (4*R*)-hydroxy-(2*S*) N-{4-[(3'-carboxy butyl)oxy]-5-methoxy-2-nitrobenzoyl}pyrrolidine-2-car-boxaldehyde diethyl thioacetal of formula II (2.58 g, 5 mmol) was taken in dry CH₂Cl₂ (20 mL), TEA (707 mg, 7 mmol) was added and the mixture was cooled at 0-5 °C. Isobutyl chloroformate (819 mg, 6 mmol) in dry CH₂Cl₂ (10 mL) was added dropwise and the mixture was kept at 0-5 °C for 15 min. A solution of 1-amino pyrene of formula I (251 mg, 5 mmol) in CH₂Cl₂ was added to it at the same temperature and the solution was stirred at room temperature for overnight. The mixture was washed with saturated NaHCO₃ (50 mL), brine, dried and solvent was evaporated. The crude material was chromatographed over silica gel using ethyl acetate/hexane (8:2) solvent to give compound (4*R*)-hydroxy-(2*S*)-N-{4-[N-(1"-pyrenyl)-butane-3'-carboxamide]-oxy-5-methoxy-2-nitrobenzoyl}pyrrolidine-2-carboxaldehyde diethyl thioacetal of formula III as a yellow liquid.

The nitro diethyl thioacetal (4*R*)-hydroxy-(2*S*)-N-{4-[N-(1"-pyrenyl)-butane-3'-carboxamide]-oxy-5-methoxy-2-nitrobenzo-yl}pyrrolidine-2-carboxaldehyde diethyl thioacetal of formula III (0.715 g, 1 mmol) was dissolved in ethyl acetate (15 mL) and added SnCl₂.2H₂O (1.12 g, 5 mmol) was refluxed for 3 h or until the TLC indicated that reaction was completed. The reaction mixture was then adjusted to pH 8 carefully with saturated NaHCO₃ solution, diluted with ethyl acetate, filtered through celite and extracted. The combined organic phase was dried over Na₂SO₄, and evaporated under vacuum to afford the crude amino diethyl thioacetal ((4*R*)-hydroxy-(2*S*)-N-{4-[N-(1"-pyrenyl)-butane-3'-carboxamide]-oxy-5-methoxy-2-aminobenzoyl}pyrrolidine-2-carboxaldehyde diethyl thioacetal of formula IV.

A solution of (4*R*)-hydroxy-(2*S*)-N-{4-[N-(1"-pyrenyl)-butane-3'-carboxamide]-oxy-5-methoxy-2-aminobenzoyl}pyrrolidine-2-carboxaldehyde diethyl thioacetal of formula IV (685 mg, 1 mmol), HgCl₂ (613 mg, 2.26 mmol) and CaCO₃ (246 mg, 2.46 mmol) in MeCN-water (4:1) was stirred slowly at room temperature until TLC indicates complete loss of starting material. The reaction mixture was diluted with EtOAc (30 mL) and filtered through a celite bed. The clear yellow organic supernatant was extracted with saturated 5% NaHCO₃ (20 mL), brine (20 mL) and the combined organic phase is dried (Na₂SO₄). The organic layer was evaporated in vacuum and purified by column chromatography (90% CH₂Cl₂-MeOH) to give compound 7-Methoxy-8-[N-(1"-pyrenyl)-butne-4'-carboxamide]-oxy-(4*R*)-hydroxy-(11a*S*)-1,2,3,11a-tetra-hydro-5*H*-pyrrolo[2,1-*c*][1,4]benzodiazepin-5-one of formula V as pale yellow oil.

### Biological Activity:

*In vitro* biological activity studies were carried out at National Cancer Institute (USA).

### Cytotoxicity:

Compounds Ve and Vg were evaluated the primary anti-cancer activity **(Table 1)** and further Ve have been evaluated in vitro against sixty human tumour cells derived from nine cancer types (leukemia, non-small-cell lung, colon, CNS, melanoma, ovarian, prostate, and breast cancer). For each compound, dose response curves for each cell line were measured at a minimum of five concentrations at 10 fold dilutions. A protocol of 48 h continuous drug exposure was used and a sulforhodamine B (SRB) protein assay was used to estimate cell viability or growth. The concentration causing 50% cell growth inhibition (GI50), total cell growth inhibition (TGI 0% growth) and 50% cell death (LC50, -50% growth) compared with the control was calculated. The mean graph midpoint values of log₁₀TGI and log₁₀LC50 as well as log₁₀ GI50 for Ve are listed in **Table 2.** As demonstrated by mean graph pattern, compound Ve exhibits an interesting profile of activity and selectivity for various cell lines.
The mean graph mid point of log₁₀ TGI and log₁₀ LC50 showed similar pattern to the log₁₀ GI50 mean graph mid points.

**Table 1. In vitro one dose primary anticancer assay^{a} pyrene linked PBD hybrid of formula Ve and Vg**

| PBD hybrids | Growth percentages | | |
|---|---|---|---|
| | (Lung) NCI-H460 | (Breast) MCF7 | (CNS) SF-268 |
| **Ve** | 11 | 31 | 70 |
| **Vg** | 106 | 72 | 131 |

| | | | |
|---|---|---|---|
| ^{a}One dose of **Ve** and **Vg** at 10⁻⁴ molar concentration | | | |

The novel pyrrolobenzodiazepine hybrid formula VIIa has shown to possess 10 nano molar potency (at the LC₅₀ level) against one non-small cell lung cancer (NCI-H226) and one colon cancer (HCC-2998). and 0.1 micro molar potency against leukemia cancer (SR), melanoma cancer (M14), renal cancer (A498) and CNS cancer (SF-539) and also have 10 micro molar potency against two CNS cancer cell lines (SF539, SNB75) and one prostate cancer (DU-145). The LC50 values of nine cancers (average of six to nine canoer cell lines) of compound VIIa listed in **Table 3**

**Table 2. log₁₀ GI50 log₁₀ TGI and log₁₀ LC50 mean graphs midpoints(MG_MID) of in vitro cytotoxicity data for the compound Ve against human tumour cell lines.**

| **Compound** | **Log₁₀ GI50** | **Log₁₀ TGI** | **Log₁₀ LC50** |
|---|---|---|---|
| **Ve** | -7.75 | -6.89 | -4.74 |

**Table 3. Log LC50 (concentration in mol/L causing 50% lethality) Values for Compounds Ve**

| **Cancer** | **Compound** **Ve** |
|---|---|
| Leukemia | -4.65 |
| Non-small-cell lung | -4.67 |
| Colon | -5.00 |
| CNS | -5.23 |
| Melanoma | -5.62 |
| Ovarian | > -4.00 |
| Renal | -5.05 |
| Prostate | -5.30 |
| Breast | > -4.00 |

Each cancer type represents the average of six to nine different cancer cell lines.

## Claims

1. Pyrrolo[2,1-*c*][1,4]benzodiazepine hybrid of formula V wherein R is H, OH and n is 1-4.

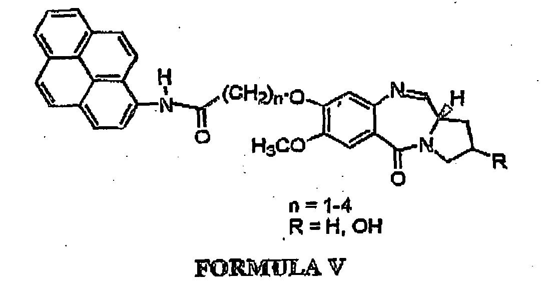

2. A pyrrolobenzodiazepine hybrid as claimed in claim 1 of the structural formula

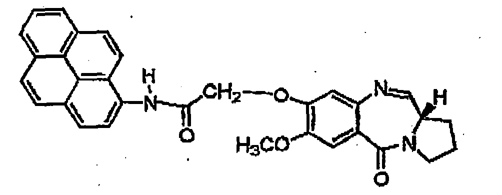

3. A pyrrolobenzodiazepine hybrid as claimed in claim 1 of the structural formula

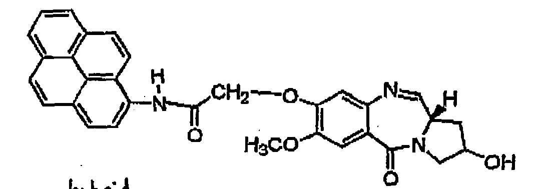

4. A pyrrolobenzodiazepine hybrid as claimed in claim 1 of the structural formula

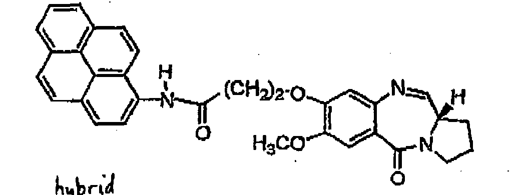

5. A pyrrolobenzodiazepine hybrid as claimed in claim 1 of the structural formula

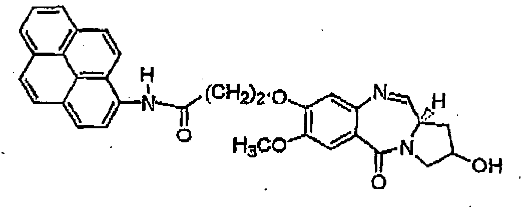

6. A pyrrolobenzodiazepine hybrid as claimed in claim 1 of the structural formula

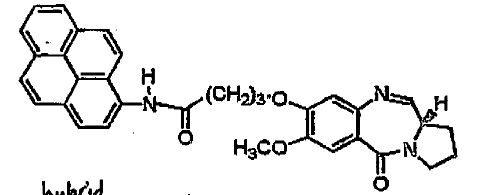

7. A pyrrolobenzodiazepine hybrid as claimed in claim 1 of the structural formula

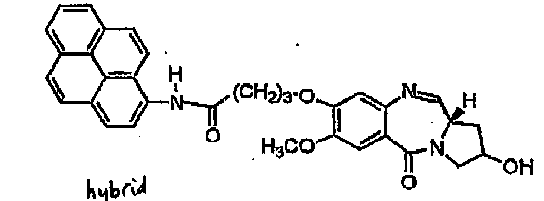

8. A pyrrolobenzodiazepine hybrid as claimed in claim 1 of the structural formula

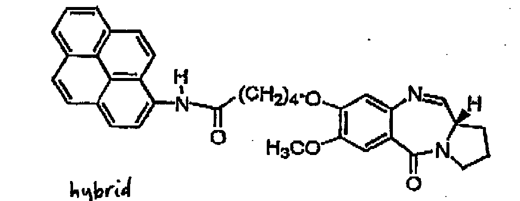

9. A pyrrolobenzodiazepine hybrid as claimed in claim 1 of the structural formula

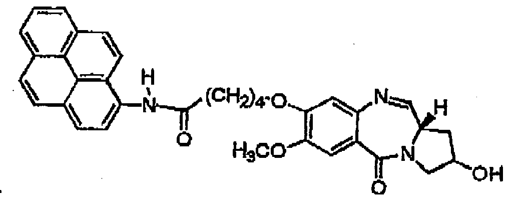

10. A process for preparing a pyrrolo[2,1-*c*][1,4]benzodiazepine hybrid of formula V 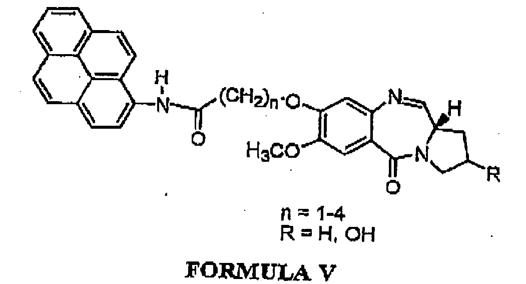 which comprises reacting pyrene amine of formula I 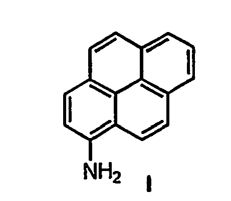 with (2*S*)-N-{4-[(3'-carboxy alkyl)oxy]-5-methoxy-2-nitrobenzoyl}pyrrolidine-2-carboxaldehyde diethyl thioacetal of formula II where R is as stated above 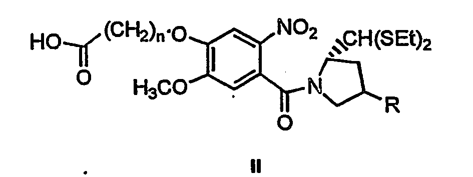 up to refluxing for a period of 24 h isolating (2*S*)-N-{4-[N-(1"-pyrenyl)-alkane-3'-carboxamide]-oxy--5-methoxy-2-nitrobenzoyl}pyrrolidine-2-carboxaldehyde diethyl thioacetal III where n is 1-4 and R is as stated above, 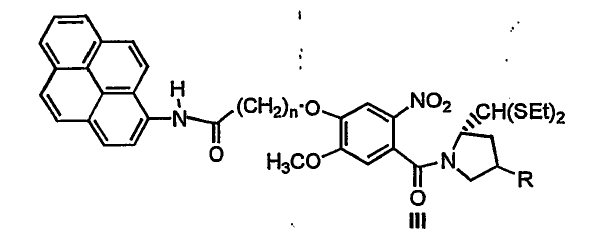 reducing the nitro compounds of formula III with SnCl₂.2H₂O in presence of an organic solvent up to a reflux temperature, isolating the (2*S*)-N-{4-[N-(1"-pyrenyl)-alkane-3'-carboxamide]-oxy--5-methoxy-2-aminobenzoyl}pyrrolidine-2-carboxaldehyde diethyl thioacetal of formula IV where n is 1-4 and R is as stated above, 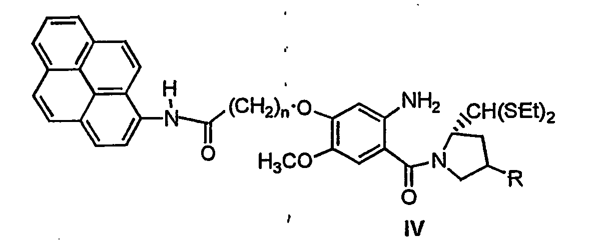 reacting the amino compound of formula IV with a deprotecting agent to give pyrrolo [2,1-*c*][1,4]benzodiazepine hybrids of formula V wherein n and R are as stated above.

11. A process as claimed in claim 10 wherein the reaction between the compound of formula I and the compound of formula II carried out in in the presence of isobutyl chloroformate and in the presence of a base selected from the group consisting of triethyl amine and DBU: and in the presence of an organic solvent selected from the group consisting of ethyl acetate, hexane and dichloromethane.

12. A process as claimed in claim 10 wherein the organic solvent used for the reduction of the nitro compound of formula III comprises ethyl acetate.

13. A pyrrolo [2,1-C] [1,4] benzodiazepine hybrid as claimed in any of claims 1-9, for use in the treatment of cancer.

14. A pyrrolo [2,1-C] [1,4] benzodiazepine hybrid as claimed in any of claims 1-9, for use in the treatment of leukaemia, non-small-cell lung, colon, CNS, melanoma, ovarian, prostate or breast cancer.

15. The use of a pyrrolo [2,1-C] [1,4] benzodiazepine hybrid as claimed in any of claims 1-9, in the manufacture of a medicament for the treatment of cancer.

16. The use according to claim 15, wherein the cancer is leukaemia, non-small-cell lung, colon, CNS, melanoma, ovarian, prostate or breast cancer.

## Patentansprüche

1. Pyrrolo[2,1-*c*][1,4]benzodiazepin-Hybrid der Formel V, worin R für H, OH und n für 1-4 steht.

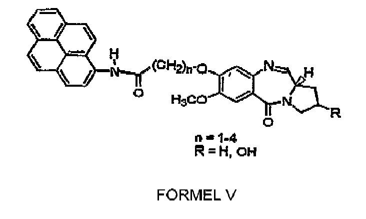

2. Pyrrolobenzodiazepin-Hybrid nach Anspruch 1 der Strukturformel

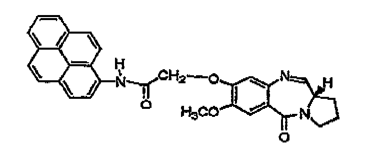

3. Pyrrolobenzodiazepin-Hybrid nach Anspruch 1 der Strukturformel

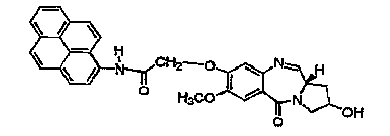

4. Pyrrolobenzodiazepin-Hybrid nach Anspruch 1 der Strukturformel

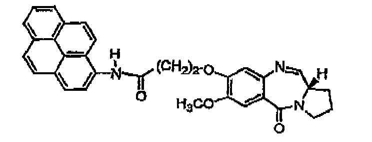

5. Pyrrolobenzodiazepin-Hybrid nach Anspruch 1 der Strukturformel

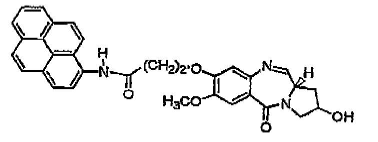

6. Pyrrolobenzodiazepin-Hybrid nach Anspruch 1 der Strukturformel

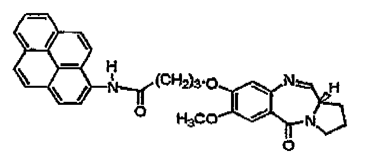

7. Pyrrolobenzodiazepin-Hybrid nach Anspruch 1 der Strukturformel

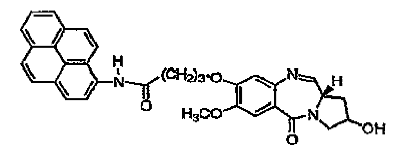

8. Pyrrolobenzodiazepin-Hybrid nach Anspruch 1 der Strukturformel

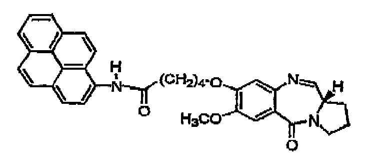

9. Pyrrolobenzodiazepin-Hybrid nach Anspruch 1 der Strukturformel

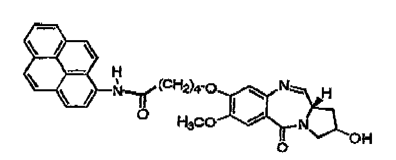

10. Verfahren zur Herstellung eines Pyrrolo[2,1-c][1,4]benzodiazepin-Hybrids der Formel V: 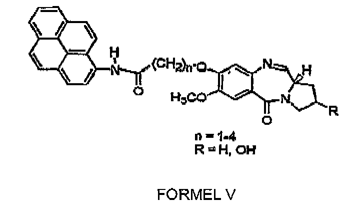 umfassend zur Reaktion bringen von Pyrenamin der Formel I 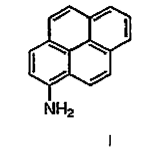 mit (2S)-N-{4-[(3'-Carboxyalkyl)oxy]-5-methoxy-2-nitrobenzoyl}pyrrolidin-2-carboxaldehyd-diethylthioacetal der Formel II, worin R wie vorstehend angegeben ist, 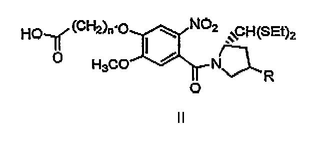 bis zum Rückfluss für eine Zeitdauer von 24 h, wobei (2S)-N-{4-[N-(1 "-Pyrenyl)-alkan-3'-carboxamid]oxy-5-methoxy-2-nitrobenzoyl}pyrrolidin-2-carboxaldehyd-diethylthioacetal III isoliert wird, worin n für 1-4 steht und R ist wie vorstehend angegeben, 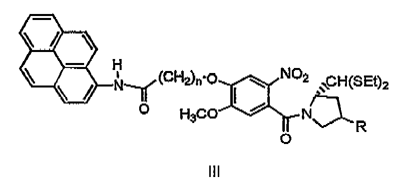 Reduzieren der Nitroverbindungen der Formel III mit SnCl₂-2H₂O in Anwesenheit eines organischen Lösungsmittels bis zu einer Rückflusstemperatur, wobei das (2S)-N-(4-[N-(1"-Pyrenyl)-alkan-3'-carboxamid]-oxy-5-methoxy-2-aminobenzoyl}pyrrolidin-2-carboxaldehyd-diethylthioacetal der Formel IV isoliert wird, worin n für 1-4 steht und R ist wie vorstehend angegeben, 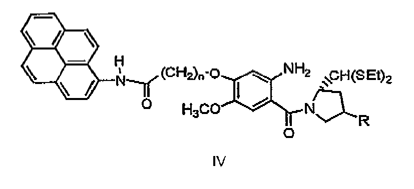 zur Reaktion bringen der Aminoverbindung der Formel IV mit einem Entschützungsmittel, um Pyrrolo[2,1-c][1,4]benzodiazepin-Hybriden der Formel V zu erhalten, wobei n und R wie vorstehend angegeben sind.

11. Verfahren nach Anspruch 10, wobei die Reaktion zwischen der Verbindung der Formel I und der Verbindung der Formel II durchgeführt wird in Anwesenheit von Isobutylchlorformiat und in Anwesenheit einer Base, die aus der Gruppe ausgewählt ist, die aus Triethylamin und DBU besteht, und in Anwesenheit eines organischen Lösungsmittels, das aus der Gruppe ausgewählt ist, die aus Ethylacetat, Hexan und Dichlormethan besteht.

12. Verfahren nach Anspruch 10, wobei das zur Reduktion der Nitroverbindung der Formel III verwendete organische Lösungsmittel Ethylacetat umfasst.

13. Pyrrolo[2,1-c][1,4]benzodiazepin-Hybrid nach einem der Ansprüche 1-9 zur Anwendung in der Behandlung von Krebs.

14. Pyrrolo[2,1-c][1,4]benzodiazepin-Hybrid nach einem der Ansprüche 1-9 zur Anwendung in der Behandlung von Leukämie, nicht kleinzelligem Lungen-, Kolon-, ZNS-, Ovarial-, Prostata- oder Brustkrebs oder einem Melanom.

15. Verwendung eines Pyrrolo[2,1-c][1,4]benzodiazepin-Hybrids nach einem der Ansprüche 1-9 in der Herstellung eines Arzneimittels zur Behandlung von Krebs.

16. Anwendung nach Anspruch 15, wobei der Krebs Leukämie, ein nicht kleinzelliger Lungen-, Kolon-, ZNS-, Ovarial-, Prostata- oder Brustkrebs oder ein Melanom ist.

## Revendications

1. Pyrrolo[2,1-c][1,4]benzodiazépine hybride de formule V, dans laquelle R est H, OH et n vaut 1-4,

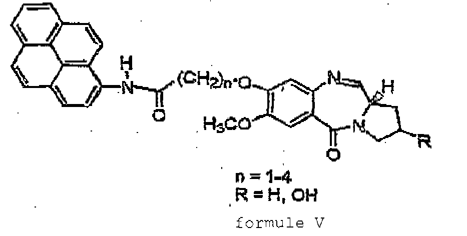

2. Pyrrolobenzodiazépine hybride selon la revendication 1, ayant la formule développée

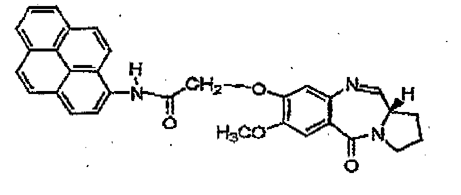

3. Pyrrolobenzodiazépine hybride selon la revendication 1, ayant la formule développée

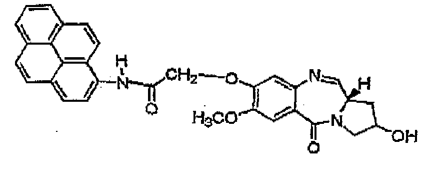

4. Pyrrolobenzodiazépine hybride selon la revendication 1, ayant la formule développée

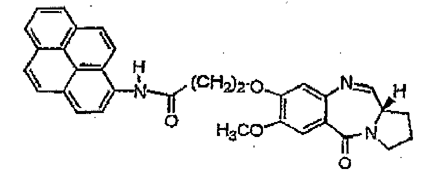

5. Pyrrolobenzodiazépine hybride selon la revendication 1, ayant la formule développée

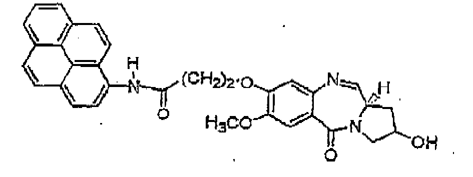

6. Pyrrolobenzodiazépine hybride selon la revendication 1, ayant la formule développée

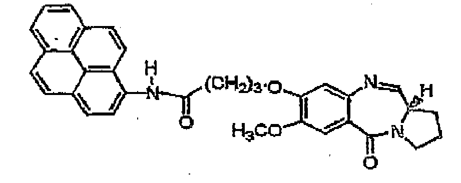

7. Pyrrolobenzodiazépine hybride selon la revendication 1, ayant la formule développée

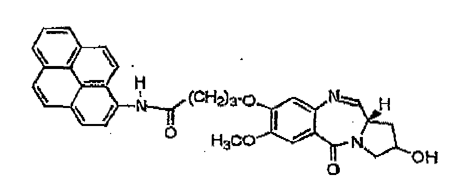

8. Pyrrolobenzodiazépine hybride selon la revendication 1, ayant la formule développée

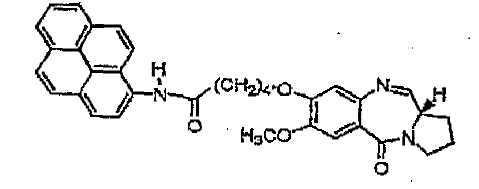

9. Pyrrolobenzodiazépine hybride selon la revendication 1, ayant la formule développée

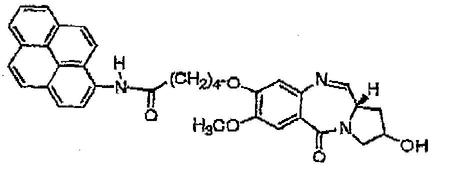

10. Procédé de préparation d'une pyrrolo[2,1-c][1,4]benzodiazépine hybride de formule V 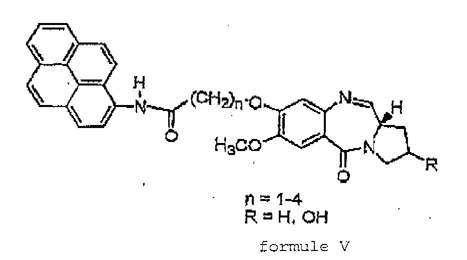 qui comprend la réaction d'une pyréne-amine de formule I 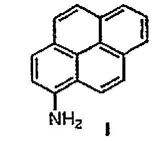 avec le thioacétal diéthylique du (2S)-N-{4-[(3'-carboxyalkyl)oxy]-5-méthoxy-2-nitrobonzoyl}pyrrolidine-2-carboxaldéhyde de formule II, dans laquelle R est tel que défini ci-dessus 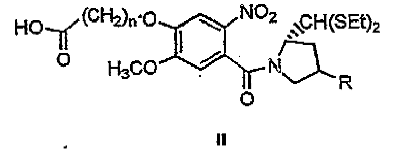 jusqu'au reflux pendant une durée de 24 h, l'isolement du thioacétal diéthylique du (2S)-N-{4-[N-(1"-pyrényl)-alcane-3'-carboxamide]-oxy-5-méthoxy-2-nitrobenzoyl}-pyrrolidine-2-carboxaldéhyde III, où n vaut. 1-4 et R est tel que défini ci-dessus, 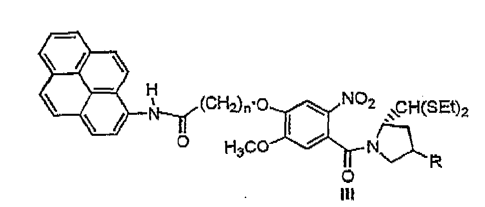 la réduction des composés nitrés de formule III avec du SnCl₂,2H₂O en présence d'un solvant organique jusqu'à une température de reflux, l'isolement du (25)-N-{4-[N-(1"-pyrényl)alcane-3'-carboxamide]-oxy-5-méthoxy-2 amino-benzoyl}pyrrolidine-2-carboxaldéhyde de formule IV dans laquelle n vaut 1-4 et R est tel que défini ci-dessus, 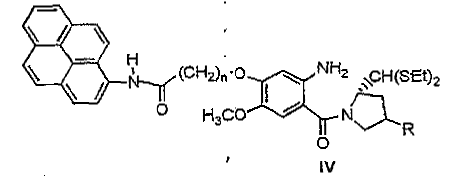 la réaction du composé aminé de formule IV avec un agent de déprotection, pour donner les pyrrolo[2,1-c][1,4]-benzodiazépines hybrides de formule V dans laquelle n et R sont tels que définis ci-dessus.

11. Procédé selon la revendication 10, dans lequel la réaction entre le composé de formule I et le composé de formule II est mise en oeuvre en présence de chloroformiate d'isobutyle et en présence d'une base choisie dans le groupe consistant en la triéthylamine et le DBU, et en présence d'un solvant organique choisi dans le groupe consistant en l'acétate d'éthyle, l'hexane et le dichlorométhane.

12. Procédé selon la revendication 10, dans lequel le solvant organique utilisé pour la réduction du composé nitré de formule III comprend de l'acétate d'éthyle.

13. Pyrrolo[2,1-c][1,4]benzodiazépine hybride selon l'une quelconque des revendications 1 à 9, pour utilisation dans le traitement du cancer.

14. Pyrrolo[2,1-c][1,4]benzodinzépine hybride selon l'une quelconque des revendications 1 à 9, pour utilisation dans le traitement de la leucémie, du cancer des poumons à grandes cellules, du cancer du colon, du SNC, avec présence de mélanomes, des ovaires, de la prostate ou du sein.

15. Utilisation d'une pyrrolo[2,1-c][1,4]-benzodiazépine hybride selon l'une quelconque des revendications 1 à 9 pour la préparation d'un médicament destiné au traitement du cancer.

16. Utilisation selon la revendication 15, pour laquelle le cancer est une leucémie, un cancer des poumons à grandes cellules, du SNC, en présence de mélanomes, des ovaires, de la prostate ou du sein.
